# EUROPEAN PATENT SPECIFICATION

(11) **EP 3 370 751 B1**
(45) Date of publication and mention of the grant of the patent: **19.10.2022**
(21) Application number: 16790391.3
(22) Date of filing: 03.11.2016
(51) Int. Cl.: A23L 33/105, A23L 33/125, A23L 33/22, A61K 36/48, A61P 1/00, A61P 3/00, A61P 3/04, A61P 3/06, A61P 3/10, A61P 9/10

(54) **LENTIL EXTRACT WITH CHOLESTEROL LOWERING AND PREBIOTIC ACTIVITY**
LINSENEXTRAKT MIT CHOLESTERINSENKENDER UND PRÄBIOTISCHER WIRKUNG
EXTRAIT DE LENTILLE À ACTIVITÉ HYPOCHOLESTÉROLÉMIANTE ET PRÉBIOTIQUE

(30) Priority: 05.11.2015 IT UB20154965
(43) Date of publication of application: 12.09.2018
(73) Proprietor: Universita' Degli Studi Di Camerino, 62032 Camerino (MC) (IT); Synbiotec S.r.l., 62032 Camerino (MC) (IT)
(72) Inventor: CECCHINI, Cinzia, 62018 Potenza Picena (MC) (IT); CRESCI, Alberto, 62032 Camerino (MC) (IT); SAGRATINI, Gianni, 62032 Camerino (MC) (IT); VITTORI, Sauro, 62032 Camerino (MC) (IT); CAPRIOLI, Giovanni, 62032 Camerino (MC) (IT); CIFANI, Carlo, 62032 Camerino (MC) (IT); MICIONI DI BONAVENTURA, Maria Vittoria, 62032 Camerino (MC) (IT); VILA DONAT, Pilar, 46195 Llombai, Valencia (ES)
(74) Representative: Cantaluppi, Stefano
(86) International application number: PCT/EP2016/076610
(87) International publication number: WO 2017/077004

(56) References cited:
- US-A1- 2010 056 470
- RAQUEL G. RUIZ ET AL: "Effect of Soaking and Cooking on the Saponin Content and Composition of Chickpeas ( Cicer arietinum ) and Lentils ( Lens culinaris )", JOURNAL OF AGRICULTURAL AND FOOD CHEMISTRY, vol. 44, no. 6, 1 January 1996 (1996-01-01), pages 1526-1530, XP055270558, US ISSN: 0021-8561, DOI: 10.1021/jf950721v
- RAQUEL GEMA RUIZ ET AL: "A preliminary study on the effect of germination on saponin content and composition of lentils and chickpeas", ZEITSCHRIFT FUER LEBENSMITTELUNTERSUCHUNG UND -FORSCHUNG., vol. 203, no. 4, 1 July 1996 (1996-07-01), pages 366-369, XP055270548, XX ISSN: 0044-3026, DOI: 10.1007/BF01231075
- A K SAMANTA ET AL: "Prebiotics in ancient Indian diets", CURRENT SCIENCE., vol. 101, no. 10, 1 July 2011 (2011-07-01) , pages 43-46, XP055270682, IN ISSN: 0011-3891
- NICOLA LANDI ET AL: "Valle Agricola lentil, an unknown lentil (Lens culinaris Medik.) seed from Southern Italy as a novel antioxidant and prebiotic source", FOOD & FUNCTION, vol. 6, no. 9, 1 January 2015 (2015-01-01) , pages 3155-3164, XP055270683, GB ISSN: 2042-6496, DOI: 10.1039/C5FO00604J
- FARIS M A I E ET AL: "Role of lentils (Lens culinaris L.) in human health and nutrition: a review", MEDITERRANEAN JOURNAL OF NUTRITION AND METABOLISM 2013 DEPARTMENT OF CLINICAL NUTRITION, COLLEGE OF APPLIED MEDICAL SCIENCES, UNIVERSITY OF HAIL, P.O. BOX 2440, HAIL, SAUDI ARABIA. TEL. +966-5-49847886. FAX +966-6-5316982. E-MAIL MOEZFUPSILONOP.EDU.J, vol. 6, no. 1, 1 January 2013 (2013-01-01) , pages 3-16, XP009189932, ISSN: 1973-798X
- LEE S-O ET AL: "Soyasaponins lowered plasma cholesterol and increased fecal bile acids in female golden Syrian hamsters", EXPERIMENTAL BIOLOGY AND MEDICINE, SOCIETY FOR EXPERIMENTAL BIOLOGY AND MEDICINE, vol. 230, no. 7, 1 July 2005 (2005-07-01), pages 472-478, XP009189920, ISSN: 1535-3702
- RAQUEL G RUIZ ET AL: "Effect of seed size and testa colour on saponin content of Spanish lentil seed", FOOD CHEMISTRY, vol. 58, no. 3, 1 January 1997 (1997-01-01), pages 223-226, XP055270553, NL ISSN: 0308-8146

## Description

### FIELD OF THE INVENTION

The present invention relates to the field of plant extracts, in particular of plant extracts with nutraceutical activity.

More in particular, the present invention relates to a lentil extract with cholesterol lowering and prebiotic effects.

### BACKGROUND OF THE INVENTION

Legumes are the edible seeds of various leguminous crops, such as lentils *(Lens culinaris* L.), beans (*Phaseolus vulgaris* L.), peas (*Pisum sativum* L.), chickpeas (*Cicer arietinum* L.), lupins (*Lupinus* spp.), broad beans (*Vicia faba* or *Faba vulgari*s)*,* soybeans (*Glycine max*), and others. Farmed for thousands of years (Messina M. J. Legumes and soybeans: overview of their nutritional profiles and health effects. Am. J. Clin. Nutr. 1997, 70, 439-450), legumes have played important roles in traditional diets in many parts of the world. They are rich in protein of vegetal origin and complex carbohydrates, which are present primarily as dietary fiber. They contain a low percentage of prevalently saturated fats and are a good source of various highly bioactive molecules and micronutrients.

In terms of nutritional and functional characteristics, lentils *(Lens culinaris* L.) are one of the most interesting legume of all. The FAO estimates world lentil production (in 2008) at around 2.83 million tons, which are grown primarily in Canada (36.9%) and India (28.7%), followed by Nepal, China, and Turkey. As regards macronutrients, lentils are a good source of protein (25.8%) composed of essential and non-essential amino acids, and certain biologically active proteins for the human organism; furthermore, a very high fraction (89%) of all the available nitrogen in lentils is contained precisely in the protein fraction of lentils. Total carbohydrates are the leading component of lentils (60.1%) with starch occupying a relevant position followed by total dietary fibers (30.5%), of which insoluble dietary fibers account for approximately 93-99.7% of all (Faris, M.A.E.; Takruri, H.R.; Issa, A.Y.; Role of lentils (Lens Culinaris L.) in human health and nutrition: a review, Mediterr. J. Nutr. Metab. 2013, 6, 3-16). Lentils are low in fat (1.1%) and for such reason have a low energy value; also worthy of notice is the fact that polyunsaturated fatty acids (PUFA, 58.8%) predominate among all fatty acids, followed by monounsaturated fatty acids (MUFA, 23.7%) and saturated fatty acids (SFA, 16.7%). As regards micronutrients, high levels of Fe, Mg, P, Ca, and S are present, with a low concentration of Na and a K:Na ratio of around 30:1-90:1. Lentils contain also vitamins such as folates, thiamin, (vitamin B1) and riboflavin (vitamin B2), in addition to niacin, pantothenic acid, and pyridoxine. Also alpha (Vitamin E), beta, and gamma tocopherol have been shown to be present in significant concentration (around 6 mg/100 g) in lentils.

Lentils are also an important source of "phytochemicals" and bioactive molecules, such as polyphenols (flavonols, tannins, and phenolic acids), phytates, phytosterols, and bioactive peptides, as well as trypsin and defensin inhibitors, isoflavones, and saponin. A study by Xu et al. published in 2007 reports that lentils show a higher concentration of polyphenols and more effective anti-oxidant capacity *in vitro* than the other legumes tested: peas, beans, and yellow soybean (B.J. Xu, S.H. Yuan, S.K.C. Chang, Comparative analyses of phenolic composition, antioxidant capacity, and color of cool season legumes and other selected food legumes, Journal of Food Science, 2007, 72, 167-177).

Lentil extracts for various therapeutic and healthy applications are known.

JP2008184440, for example, discloses a lentil extract to be applied on the skin for the mitigation and remedy of sunburn.

UA63766 discloses a lentil extract with analgesic hepatoprotective activity and a regenerative effect on the pancreas.

US5762936 discloses an extract obtained from the hulls of lentil seeds enriched with polyphenols characterized by antioxidant activity against free radicals. The extract can be used as an anti-oxidant supplement to prevent or improve chronic inflammation and pathologies caused by free radicals.

A growing body of scientific evidences reports that legumes in general and lentils in particular possess cardioprotective, hypolipidemic, and homocysteine lowering effects. More specifically, epidemiologic studies have shown the consumption of legumes and lentils to be inversely linked to the risk of contracting cardiovascular disease, type 2 diabetes, and obesity, lower LDL cholesterol levels, and higher HDL cholesterol levels.

Hypercholesterolemia has been shown to be one of the leading risk factors behind serious cardiovascular events such as acute myocardial infarction. Furthermore, a close link has been observed between these disorders and abnormal increases in lipids, especially high levels of plasma cholesterol and high blood pressure as a result.

As mentioned above, the beneficial properties of lentils have been ascribed to the presence of bioactive molecules, such as saponins, for example. Lentils are an excellent dietary source of saponins, the triterpenoidal or steroidic structured glycosides naturally present in plants and considered components of dietary fiber. The saponins in lentils, commonly known as soyasaponin because they are also present in soybeans, have been shown to possess many health properties, most notable of which are the reduction of plasma cholesterol levels, anti-carcinogenic and anti-hepatotoxic effects, and anti-replicative effects against the HIV virus. Scientific evidence shows that dietary saponins are capable of lowering plasma cholesterol levels by means of a mechanism that inhibits the absorption of exogenous cholesterol by the small intestine or through an indirect bile acid absorption inhibition mechanism. Lentil soyasaponins are triterpenoidal glycosides that are structurally divided in two groups, one known as A (bidesmosidic), with two potential glycosilation sites, the other known as B (monodesmosidic), with only one glycosilation site. Lentils contain primarily soyasaponin I (soyasaponin beta b) and soyasaponin *β*g (also referred to as soyasaponin VI), both of which are members of the soyasaponin B group. Soyasaponin *β*g contains the DDMP group (5-hydroxy-6-methyl-2H-pyran-4(3H)-one) at the C-22 position and may be considered the natural precursor of soyasaponin I (Sagratini G., Zuo Y, Caprioli G., Cristalli G., Giardinà D., Maggi F, Molin L., Ricciutelli M., Traldi P., Vittori S. Quantification of Soyasaponins I and βg in Italian Lentil Seeds by Solid Phase Extraction (SPE) and High Performance Liquid Chromatography-Mass Spectrometry (HPLC-MS). J. Agric. Food Chem. 2009, 57, 11226-11233). In the works of Raquel G. Ruiz Et Al ("Effect of Soaking and Cooking on the Saponin Content and Composition of Chickpeas (Cicer arietinum) and Lentils (Lens culinaris)", JOURNAL OF AGRICULTURAL AND FOOD CHEMISTRY. vol. 44. no.6. 1 January 1996, pages 1526-1530, and "A preliminary study on the effect of germination on saponin content and composition of lentils and chickpeas", ZEITSCHRIFT FUER LEBENSMITTELUNTERSUCHUNG UNO - FORSCHUNG, vol. 203. no.4. 1 July 1996 (1996-07-01), pages 366-369) saponin content in lentils is investigated; saponins are extracted from lentils using aqueous ethanol containing ethylenediaminetetraacetic acid (EDTA).

With regard to the cholesterol lowering activity of saponins, some studies, such as a review by Oakenfull et al. (Oakenfull, D.; Sidhu, G.S. Could saponins be a useful treatment for hypocholesterolaemia? Europ. J. Clin. Nutr., 1990, 44, 79-88), report the results of one study conducted with 174 persons given a saponin-rich diet in which convincing (around 20%) reductions in plasma cholesterol were observed in some cases. This review also mentioned an *in vivo* study on the use of concentrated saponin extracts in which substantial reductions in plasma and hepatic cholesterol were reported. The authors conclude that there is substantial evidence that dietary saponins can lower cholesterol levels. Lee et al. refer to an *in vivo* study on golden Syrian hamsters fed a diet rich in soyasaponin in which reductions in both total and LDL cholesterol was observed compared to others fed a diet rich in caseine by means of a mechanism that provokes greater expulsion of bile acid and neutral sterols (Lee, S.O.; Simons, A.L.; Murphy, P.A.; Hendrich, S. Soyasaponins lowered plasma cholesterol and increased fecal bile acids in female golden Syrian hamsters. Exp. Bio. Med. 2005, 230, 472-478).

However, no hypocholesterolemic activity of a lentil extract, when administered to a subject, has been shown.

Prebiotics are defined as "dietary supplements or ingredients with the capacity to stimulate the growth and/or activity of one or a limited number of species of microbes present in intestinal microbiota to the benefit of the health of the host" (Roberfroid, M.; Gibson, G.R.; Hoyles, L.; McCartney, A.L.; Rastall, R.; Rowland, I.; Wolvers, D.; Watzl, B.; Szajewska, H.; Stahl, B.; Guarner, F.; Respondek, F.; Whelan, K.; Coxam, V.; Davicco, M.J.; Léotoing, L.; Wittrant, Y.; Delzenne, N.M.; Cani, P.D.; Neyrinck, A.M.; Meheust, A. Prebiotic effects: metabolic and health benefits. Br. J Nutr. 2010, 104 Suppl 2:S1-63). Most known prebiotics are indigestible galacto-oligosaccharides (GOS) and fructo-oligosaccharides (FOS) such as inulin and oligofructose. Several dietary fibers including non-starch polysaccharides, cellulose, dextrans , chitins, pectins, beta-glucans and waxes have been shown to provide beneficial effects similar to those of inulin (Napolitano, A.; Costabile, A.; Martin-Pelaez, S.; Vitaglione, P. et al., Potential prebiotic activity of oligosaccharides obtained by enzymatic conversion of durum wheat insoluble dietary fibre into soluble dietary fibre. Nutr. Metab. Cardiovasc. Dis. 2009, 19, 283-290).

Lentils are rich in FOS (fructo-oligosaccharides), among which kestose and nystose, in GOS (galacto-oligosaccharides), among which stachyose and verbascose, and in sugar alcohols, such as starch-resistant mannitol and sorbitol. A recent study by Johnson et al. reports that lentils are capable of supplying 13 g of prebiotic carbohydrates per 100 g of product, presenting a physiological activity of this legume that has not yet been explored (Casey, R.J.; Thavarajah, D.; Combs, G.F.; Thavarajah, P. Lentil (Lens culinaris L.): A prebiotic-rich whole food legume. Food Res. Int. 2013, 51, 107-113).

However, it is not present in the state of the art any evidence regarding the prebiotic effect of lentil extracts and their *in vivo* activity, which is yet unknown.

It is known that the use of prebiotics has positive effects on the health and wellbeing of the organism.

WO2012108830, for example, discloses symbiotic combinations for intestinal microbiota reconstitution comprising bacterial strains and prebiotics, wherein at least one prebiotic substance is a starch.

WO2009152089 describes a prebiotic composition for the treatment of gastrointestinal diseases. The composition comprises hemicellulose material extracted from a lignocellulosic source as a prebiotic.

Thanks in particular to their rebalancing effect on intestinal flora, prebiotics are useful in preventing and/or treating gastrointestinal disorders associated with an alteration of the intestinal flora.

Indeed, prebiotics are utilized by the intestinal microbial population to produce short-chain fatty acids which may lead to the reduced incidence of gastrointestinal diseases and other effects and in particular an improvement of lipid profiles.

Furthermore, prebiotics have also gained increasing attention in cholesterol studies due to their role in promoting the growth of "beneficial microbes", the so called probiotics that had well documented hypocholesterolemic effect. Some studies, conducted using inulin as prebiotic, demonstrated the positive effect on reducing the plasma total cholesterol and triacylglycerol concentration on humans (Brighenti, F.; Casiraghi, M.C.; Canzi, E.; Ferrari, A. Effect of Consumption of a ready-to-eat breakfast cereal containing inulin on the intestinal milieu and blood lipids in healthy male volunteers. Eur. J. Clin. Nutr. 1999, 53, 726-733).

Prebiotics thus represent a very interesting food ingredient for the development of dietary supplements and functional foods.

In view of the above, compounds capable of exerting prebiotic effects are highly wanted and desirable.

In particular, the stimulation of some species of microbes, such as Bifidobacteria, is particularly desirable for the beneficial effects that these species have been proven to exert on the organism.

### OBJECT OF THE INVENTION

The inventors of the present invention have now found a lentil extract that is surprisingly characterized, at the same time, by a significant cholesterol lowering effect and high prebiotic activity.

These characteristics make this extract particularly indicated for both therapeutic use and as a nutraceutical thanks to its beneficial activity both on the intestinal microbiota and on the cholesterol level. Moreover, it has been found that administration of the lentil extract according to the invention provides a synergic effect of the prebiotic and hypocholesterolemic activities resulting in a powerful lowering of the cholesterol level.

A lentil *(Lens culinaris* L.) extract characterized by the presence of soyasaponin I in the range comprised between 50 and 300 mg kg⁻¹ and of soyasaponin βg in the range comprised between 0.5 and 5 mg kg⁻¹ is obtained by a process characterized by the following steps:
a) adding a 70:30 mixture of water and ethanol to a grinded lentil *(Lens culinaris* L.) sample, with the proviso that said mixture does not comprise ethylenediaminetetraacetic acid (EDTA);
b) stirring the obtained mixture;
c) filtering the obtained mixture;
d) concentrating the obtained mixture until complete evaporation of ethanol.

Optionally, the process above comprises a further concentration step and the subsequent freeze-drying of the obtained mixture.

The lyophilized extract obtained with the process above is also an object of the present invention.

The lentil extract of the invention has the advantages, when consumed, of exerting the effect of lowering the total plasma cholesterol level with simultaneous prebiotic action, in this way providing the organism with numerous health benefits, e.g. intestinal health. Furthermore, the beneficial flora stimulated by the prebiotic effect of the extract (in particular, Lactobacilli and Bifidobacteria) contributes to the lowering effect on cholesterol level thus providing a synergic effect on the lowering of the cholesterol.

It is also disclosed the use of the extract, according to the invention, in the treatment or in the prevention of diseases and conditions in which a cholesterol lowering activity is desirable.

It is also an object of the present invention the extract according to the invention for use as a prebiotic, in particular as a stimulant of Bifidobacteria and/or Lactobacilli, for the preservation and/or restoration of intestinal bacterial flora.

A nutraceutical, a functional food or a dietary supplement comprising the extract or the lyophilized extract according to the invention are also objects of the present invention.

A pharmaceutical composition comprising the extract or the lyophilized extract according to the invention is a further object of the present invention.

### DETAILED DESCRIPTION OF THE INVENTION

### Definitions

In the context of the present invention, the term "extract" means a preparation, usually a concentrate, obtained from plant material.

In the context of the present invention, the term "lyophilized" or "lyophilized extract" means a preparation obtained through a process of lyophilization by means of which the water contained in the preparation is eliminated.

In the context of the present invention, the term "nutraceutical" means a preparation supposed to have a function beneficial to human health.

In the context of the present invention, the term "functional food" or "pharma food" or "alicament", means a food that contains molecules with beneficial and protective properties for the organism that help to prevent health disorders and imbalances when included in a balanced diet.

In the context of the present invention, the term "dietary supplement" means a product to be consumed in addition to a regular diet that is formulated to favor the assimilation of determined nutrients or to supplement an incorrect, unbalanced, or insufficient diet.

### Brief description of the Figures

Figure 1: Total count of Bifidobacteria for the entire duration of the fermentation (after 24, 48, 72, and 96 hours of semi-continuous fermentation) of the Control Group, the Inulin Positive Control Group, and the Lentil Extract Group.
Figure 2. Level of bile acids excreted by feces analyzed by HPLC-MS.

The lentil extract of the invention is characterized by the presence of soyasaponin I in the concentration range comprised between 50 and 300 mg kg⁻¹ and of soyasaponin βg in the concentration range comprised between 0.5 and 5 mg kg⁻¹.

Preferably, soyasaponin I is present in the extract in a concentration range comprised between 80 and 200 mg kg⁻¹, more preferably between 100 and 150 mg kg⁻¹. Preferably, soyasaponin βg is present in the extract in a concentration range comprised between 0.5 and 2 mg kg⁻¹, more preferably between 0.8 and 1 mg kg⁻¹.

It is disclosed an extract comprising soyasaponin I in a concentration of about 103 mg kg⁻¹ and soyasaponin βg in a concentration of about 0.96 mg kg⁻¹.

The extract object of the present invention is obtained from lentil seeds. In particular said seed are of the *Lens culinaris* L. species.

The lentils used can be of any sub-specie, in particular they can be of the sub-species *Lens culinaris subsp. culinaris* (farmed lentils), *Lens culinaris subsp. Odemensis, Lens culinaris subsp. Orientalis, Lens culinaris subsp. Tomentosus.* Preferably, they are lentils of the *Lens culinaris subsp. culinaris* (farmed lentils) subspecie.

The lentils can be of any cultivar, such as, for example, Castelluccio di Norcia lentil (IGP, DOP), Colfiorito lentil, Santo Stefano di Sessanio lentil, and Onano lentil. Preferred varieties are Colfiorito lentil and Castelluccio di Norcia lentil.

After the lentils seeds have been finely ground, a mixture of water and ethanol is added (Step a). Preferably, water and ethanol are present in said mixture in a water:ethanol ratio comprised between 20:80 and 80:20. For example, said water:ethanol ratio can be 20:80, 30:70, 50:50, 70:30 or 80:20. Preferably, said water:ethanol ratio is 30:70.

The mix is then stirred (Step b), for example for a period comprised between 1 and 6 hours, preferably for 3 hours.

The mix is then filtered (Step c), preferably under vacuum.

The solution thus obtained is concentrated until the complete evaporation of the ethanol. Evaporation is preferably performed in a rotary evaporator, preferably at a temperature of less than 30 °C.

An extract object of the present invention is thus obtained.

Optionally, the extract thus obtained can be further concentrated and then lyophilized until a powder is obtained using methods known in the field.

Said lyophilized extract is also an object of the present invention.

Said lyophilized extract can be, for example, in the form of a pill or a tablet and appropriately mixed with suitable excipients.

The extract or lyophilized extract according to the invention can be mixed with compounds or extracts with known cholesterol lowering activity, such as for example, phytostanols, phytosterols, fermented red rice, berberine, silymarin, and/or compounds or extracts with prebiotic activity, such as inulin, fructo-oligosaccharides (FOS), pectins, and similar substances.

In a preferred embodiment of the invention, finely ground lentils of Colfiorito variety are extracted by magnetic stirring for 3 hours, together with a mixture of water and ethanol (about 30:70); the mixture is then filtered under vacuum and subjected to evaporation until the ethanol evaporates completely.

The lentil extract described above offers the advantages when consumed of exerting the effect of lowering the total plasma cholesterol level with simultaneous prebiotic action, in this way providing the organism with numerous benefits. It has also been found that when said extract is administered it increases the level of beneficial microbial intestinal flora, for example probiotics, able to exert hypocholesterolemic effects. Therefore, a powerful effect on the lowering of the cholesterol level is provided by said extract.

By virtue of these characteristics, the extract according to the invention can be used in all cases in which a cholesterol lowering activity is desired. In particular, it can be used for the treatment of a subject suffering from a disorder associated with hypercholesterolemia.

For example, the extract can be used in the treatment of cardiovascular diseases, such as myocardial infarction, type 2 diabetes, obesity, high blood pressure, and similar disorders.

Also, it can be used in all conditions wherein a maintenance of the cholesterol level is desired. For example, it can be used in conditions wherein a subject is at risk of developing a pathology related to high cholesterol levels. For example it can be used in a subject predisposed to develop hypercholesterolemia. Also, it can be used to prevent the development of diseases related to high cholesterol levels.

For maintenance of cholesterol level is intended maintaining the cholesterol level within the limits considered acceptable for the health of a subject. A person skilled in the art, for example a physician, knows the limits considered acceptable based on the general knowledge in the field and on the conditions of the subject.

Using his general knowledge in the field, a person skilled in the art is capable of evaluating the most appropriate quantities, methods, and times required for treatment on the basis of the patient's specific conditions.

Thanks to its prebiotic properties, the extract according to the invention can be administered also as a prebiotic, in particular in order to favour beneficial gut microbes and their beneficial activities.

Prebiotics are compounds that promote the growth and activity of species of beneficial bacteria that play important roles in maintaining the health of the host. Such species are, in particular, Bifidobacteria and Lactobacilli.

The extract according to the present invention can therefore be used in conditions wherein the use of prebiotics is recommended. Such conditions are conditions which can benefit from administration of prebiotics.

Many conditions and diseases are known in the art which can benefit from the administration of prebiotics. In particular, these are conditions and diseases wherein it is known that stimulation of the growth and/or activity of some microbial species present in intestinal microbiota is beneficial.

More in particular, the extract described herein can be advantageously used for the preservation and/or restoration of intestinal bacterial flora.

The alteration of the equilibrium of the intestinal bacterial flora has been associated with the manifestation and presence of various pathologies of intestinal and other origin. In particular, intestinal dysbiosis, i.e. a microbial imbalance or maladaptation occurring in the gut, such as an impaired microbiota, is associated with the pathogenesis of both intestinal (inflammatory bowel disease, irritable bowel syndrome (IBS), and coeliac disease) and extra-intestinal disorders (allergy, asthma, metabolic syndrome, cardiovascular disease, and obesity). It is known that intake of prebiotics can significantly modulate the colonic microbiota by increasing the number of specific bacteria and thus positively changing the composition of the microbiota.

For this reason, thanks to its prebiotic activity, the extract according to the invention can be validly used in the prevention and/or treatment of gastrointestinal diseases and disorders wherein an alteration of the intestinal bacterial flora is present. For gastrointestinal disease or disorder wherein an alteration of the intestinal bacterial flora is present it is intended a condition wherein a microbial imbalance is present in the gut. In particular, it is intended a condition of intestinal dysbiosis. Said disorders may include, for example, irritable bowel syndrome, chronic inflammatory bowel diseases, diarrhea, dyspeptic forms, meteorism, diverticulosis, syndrome of bacterial overgrowth in the small intestine, alteration of the immune system, and food allergies.

Furthermore, owing to their nutritional properties, the use of prebiotics is recommended for people suffering from diabetes and/or metabolic syndrome, obesity, and food allergies (Casey R. Johnson, Dil Thavarajah, Gerald F. Combs Jr., Pushparajah Thavarajah, Lentil (Lens culinaris L.): A prebiotic-rich whole food legume, Food Research International, 2013, 51, 107-113).

In particular, the extract according to the invention has Bifidogenic properties, i.e. it is able to stimulate the growth of bifidobacteria. Bifidobacteria are anaerobic bacteria, among the main components of health intestinal microflora. Bifidobacteria exert a series of beneficial effects on the health of the organism, such as the regulation of intestinal microbe homeostasis, the inhibition of harmful pathogens and bacteria that colonize or infect the intestinal mucosa, the modulation of systemic and local immune system response, the repression of procarcinogenic enzyme activity among the microbiota, the production of vitamins, and the bioconversion of a series of food compounds into bioactive molecules.

For these reasons, the use of the extract according to the invention as a prebiotic is particularly advantageous, in particular thanks to its stimulatory effect on Bifidobacteria.

The expert in the field is capable of evaluating and choosing the appropriate treatment methods to be adopted for the use of the extract according to the invention as a prebiotic, particularly for the disorders mentioned above, as required by the patient's conditions.

The extract can also be in the form of a nutraceutical, functional food, or dietary supplement.

The extract can be formulated in any way suitable on the basis of the knowledge available to an expert in the field. In particular, formulations for oral use are preferred, such as in the form of pills, tablets, granulate, powder, syrup, gelatin, suspension, and emulsion. Any other form is also within the scope of the invention.

The extract according to the invention can also be formulated as a pharmaceutical composition or as a food with medical functions using general knowledge in the field. Such pharmaceutical composition can also comprise at least one pharmaceutically acceptable vehicle or excipient such as, for example, particularly useful formulation coadjuvants, e.g. solubilising agents, dispersing agents, suspension agents, and emulsifying agents.

The extract according to the invention can also be used in combination with other compounds, in particular with dietary supplements and/or probiotics. More specifically the use of prebiotics together with probiotics is particularly advantageous since both have hypocholesterolemic activities. For "probiotics" it is intended live micro-organisms which, when administered in adequate amounts, confer a health benefit on the host. Probiotics are known in the art.

The invention is further illustrated by the following examples.

### EXAMPLES

### Example 1

### Quantification of soyasaponin in lentil samples

Highly-specific methods have been developed in our laboratories for the quantification of concentrations of molecules that, as reported in literature, appear to be the agents primarily responsible for the cholesterol lowering effect exerted by lentils (Sagratini, G.; Caprioli, G.; Maggi, F.; Font, F.; Giardinà, D.; Mañes, G.; Meca, G.; Ricciutelli, M.; Sirocchi, V.; Torregiani, E.; Vittori, E. Determination of Soyasaponins I and βg in Raw and Cooked Legumes by Solid Phase Extraction (SPE) Coupled to Liquid Chromatography (LC)-Mass Spectrometry (MS) and Assessment of Their Bioaccessibility by an in Vitro Digestion Model, Journal of Agricultural and Food Chemistry, 2013, 61, 1702-1709; Pilar Vila Donat, Giovanni Caprioli, Paolo Conti, Filippo Maggi, Massimo Ricciutelli Elisabetta Torregiani, Sauro Vittori, Gianni Sagratini, Rapid Quantification of Soyasaponins I and βg in Italian Lentils by High-Performance Liquid Chromatography (HPLC)-Tandem Mass Spectrometry (MS/MS), Food Analytical Methods, 2014, 7, 1024-1031).

These molecules are the soyasaponin I and *β*g mentioned and described above.

1 g of seed of lentils (Colfiorito lentils) were finely grounded and then extracted by magnetic stirring for 3 hours using a 10 ml mixture of water and ethanol (30:70); the mixture was then filtered under vacuum and injected directly in HPLC-MS/MS (triple quadrupole) in order to determine the concentration of soyasaponins in the lentils being studied. The chromatographic separation of the soyasaponins I and *β*g was obtained through the use of a Gemini C₁₈ (150 × 4.6 mm i.d., 5 µm, analytical column from Phenomenex, Chesire, U.K.). The mobile phase was composed of one mixture (A) of water and 0.25% acetic acid (v/v) and another mixture (B) of methanol with 0.25% acetic acid (v/v) with a flow rate of 1 ml/min in the following isocratic conditions: 20% A, 80% B. The volume injected was 20 µl; the duration of the injection was 10 min. HPLC-MS/MS analysis was performed using an Agilent 1290 Infinity series instrument and a Triple Quadrupole 6420 Agilent Technology analyzer (Santa Clara, CA) equipped with an ESI interface in negative ionization mode.

After this method was validated, it allowed us to quantify the soyasaponin I and *β*g in the samples of lentils analyzed with values that ranged from 54 to 226 mg kg⁻¹ for soyasaponin I and from 436 to 1272 mg kg⁻¹ for soyasaponin *β*g.

### Example 2

### Nutritional and further characterization of lentil extracts

### Preparation of the extract

The lentil extract was obtained though the following process: 50 g of finely ground lentils were added to a 500 ml mixture of water and absolute ethanol (70:30) and then subjected to magnetic stirring for 3 hours. The mixture was then filtered and the solution was made to evaporate in a rotary evaporator at T<30 °C until a volume of around 300 ml was obtained (and all the ethyl alcohol was completely evaporated).

After being diluted, as required, this residual watery solution known as *Extract A* was used for *in vivo* research on animals and *in vitro* research for humans in order to evaluate its prebiotic effect.

*Extract A* was also further concentrated in the rotary evaporator at room temperature, and then lyophilized in order to obtain around 4 g of powder, referred below as *Extract B,* which is characterized by its content of bioactive molecules and nutritional substances.

### Extract A

The HPLC-MS/MS method was used for the analysis of the soyasaponins in *Extract A,* utilized for *in vivo* research on animals, and provided a concentration of 102.7 mg kg⁻¹ for soyasaponin I and 0.96 mg kg⁻¹ for soyasaponin *β*g (total soyasaponin concentration of 103.66 mg kg⁻¹).

### Extract B

The nutritional analysis of the final lyophilized *Extract B* provided the following values: a) Energy value per 100 g of product: 1532 kJ or 366 Kcal; b) total carbohydrates, as presented, 67.82%; c) nitrogenous substances, as presented, 21.06%; d) fatty substances, as presented 1.13%; e) Ash, as presented, 0.60%; f) salt, < 0.01 %; g) crude fiber, as presented, 18%, h) and humidity 9.18%.

### Example 3

### Cholesterol lowering activity of the extract

In order to evaluate the cholesterol lowering activity of the lentil extract, we used an animal model in which hypercholesterolemia was induced with the use of a cholesterol-rich diet. Nineteen Sprague-Dawley male rats (Charles River, Calco, Italia) were put into individual cages in a room kept at constant temperature (20-22°C) and humidity (45-55%). Their initial body weight was 225-250 g. The rats were subjected to a 12h light/dark cycle and fed a standard diet (4RF18; Mucedola; 2.6 kcal/g) with water ad libitum for 2 weeks prior to the experiment. Their average cholesterol levels were on average 68 ± 2 mg/100 ml.

All experiments were conducted in accordance with European Directive 2010/63/UE regulating animal welfare and protection and with the Italian Legislative Decree N. 116, January 27^{th}, 1992.

After acclimatization, all rats were fed a commercially available cholesterol-rich diet (AIN-76 diet enriched with 1% cholesterol and 0.5% cholic acid, D04082702 Research Diet, New Brunswick, NJ 08901 USA). Body weight and food intake were monitored every day.

The rats were given free access to this diet for 6 weeks, after which cholesterol levels were observed to have risen significantly (247 ± 24 mg/100 ml) over their initial values (68 ± 2 mg/100 ml).

The rats were divided in two experimental groups without significant differences in body weight [F(1.17) = 0.06, p>0.05], food intake [F(1.17) = 4.03, p>0,05], plasma cholesterol values [F(1.17) = 0.00, p>0.05], triglycerides [F(1.17) = 0.05, p>0.05], HDL [F(1.17) = 1.69, p>0.05] or LDL [F(1.17) = 0.05, p>0.05].

The two experimental groups received the following treatments for 71 days:
1) Vehicle (water) (n = 9)
2) Lentil *Extract A* containing soyasaponin (4 ml of *Extract A* /24 h) (n = 10).

Blood samples were analyzed by Fioroni laboratory (San Benedetto del Tronto (AP)).

For the entire duration of treatment, body weight, food intake, (shown in Table 1) and locomotor activity (evaluated by Open field test) were unaffected by the administration of *Extract A.*

As shown in Table 1, at the end of treatment, the blood cholesterol levels of the rats fed 4 ml of *Extract A*/*24* h were significantly lower than those of the control groups [F(1.17) = 5.06, p<0.05]. Furthermore, a non-significant trend in the reduction of LDL levels [F(1.17) = 0.70, p>0.05] and in the increase of HDL levels [F(1.17) = 2.98, p>0.05] was observed.

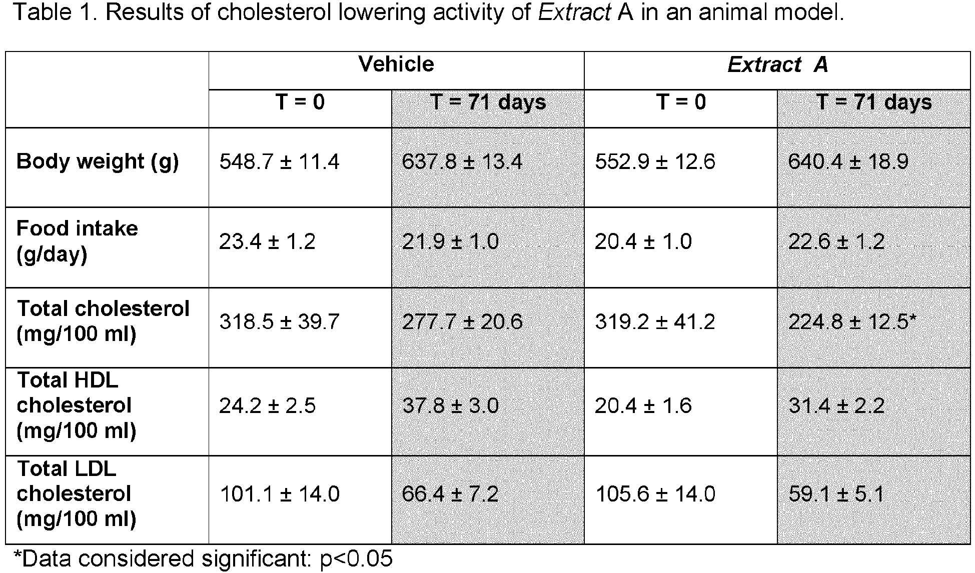

Table 1 shows that a lowering of total cholesterol levels was observed in both the rats fed with the vehicle and those fed *Extract A,* partially due to the physiological adaptation of their organisms to a cholesterol-enriched diet (exogenous), so that they were no longer capable of synthesize endogenous cholesterol at hepatic level and consequently demonstrates a physiological decrease in total cholesterol. However, after 71 days of treatment, a marked decrease in the total cholesterol level (-29.6%) of the rats fed *Extract* A was observed compared to those fed the vehicle (-12.8%). Therefore, a comparison of the decrease in the total cholesterol level of the rats treated with *Extract A* with those treated with the vehicle permits to conclude that the decrease observed in rats treated with Extract A is statistically significant (16.8%).

In LDL and HDL cholesterol levels, a trend of decrease and a trend of increase are observed respectively in rats treated with the vehicle and those treated with *Extract A,* but the differences are statistically not significant.

### Example 4

### Analysis of bile acids in feces in HPLC-MS

To confirm and to explain the mechanism involved in the hypocholesterolemic action of the lentil extract used in the *in vivo* experiment with rats, a HPLC-MS method was developed to quantify the bile acids involved in the digestion process in feces of rats. The selected bile acids were: cholic acid (CA), chenodeoxycholic acid (CDCA), deoxycholic acid (DCA), lithocholic acid (LCA), ursodeoxycholic acid (UDCA).

### Material and Methods

Freeze dried feces samples were weighed (100 mg) into a vial and 5 ml of methanol were added. Samples were vortexed for 2 min, sonicated for 30 min and then filtered through a 0.22-µm PTFE filters before HPLC analysis. HPLC-ESI-MS studies were performed using an Agilent 1290 Infinity Series and a Triple Quadrupole 6420 from Agilent Technology (Santa Clara, CA, USA) equipped with an ESI source operating in both negative and positive ionization modes. Chromatographic separation was accomplished on a Symmetry C18 (4.6 mm ×250 mm, 5 µm) analytical column. The mobile phase for HPLC-MS analyses was aqueous-formic acid (99.95-0.05%) (A) and methanol-formic acid (99.95-0.05%) (B) working in the gradient mode. The flow rate was set at 1 ml/min, and the column temperature was controlled at 30 °C. The ESI interface and mass spectrometer parameters were optimized to obtain maximum sensitivity. Mass spectra were acquired in negative polarity and acquisition was performed by using single ion monitoring (SIM) mode, by selecting the ion *m*/*z* 407 [M-H]⁻ for CA, the ion *m*/*z* 391.2 [M-H]⁻ for CDCA DCA and UDCA, the ion *m*/*z* 375.3 [M-H]⁻ for LCA.

The developed and validated method was applied to the analysis of bile acids in the feces of rats used in the previous *in vivo* study.

### Results

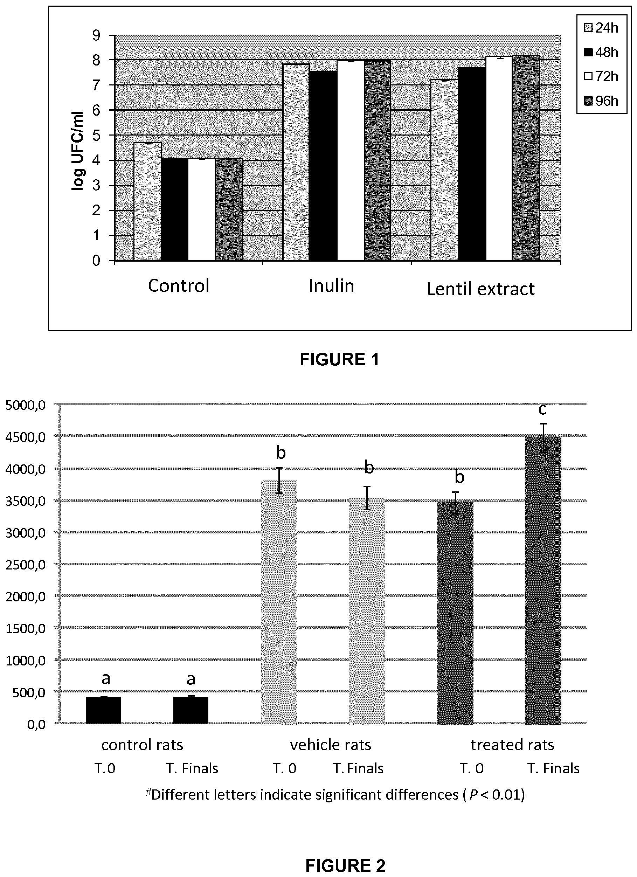

As it is shown in Figure 2, the excretion of bile acids in both control group and vehicle group, did not statistically change from the beginning (T 0) to the end of the study (T Final), as expected.

On the other side, there were significant effects of lentil extract treatment on bile acids excretion in rat feces. Mean value of total fecal bile acid was significantly higher in rats fed lentil extract (p<0.01) compared with the respective group before treatment with extract. In fact, the total amount of bile acids in feces changed from 3463.6 mg kg⁻¹ (T 0) to 4474.2 mg kg⁻¹ (T Finals) in rats treated with diluted lentil extract, with a conspicuous increase of 1010.6 mg kg⁻¹.

Thus, the cholesterol-lowering effect of lentil extract soyasaponins-rich was mediated by increased fecal output of bile acids, indicating the inhibition of their intestinal reabsorption. These data, according to a previous study realized by using different animal model and different saponins extracts (Lee, S.O.; Simons, A.L.; Murphy, P.A.; Hendrich, S. Soyasaponins lowered plasma cholesterol and increased fecal bile acids in female golden Syrian hamsters. Exp. Bio. Med. 2005, 230, 472-478), confirm the supposed mechanism to explain the hypocholesterolemic effect of lentil extract rich in soyasaponins that involves a greater excretion of bile acids. In fact, in this way, the lack of bile acids at intestinal level produces the use of endogenous cholesterol for synthesizing new bile acids, with a consequent decrease of total level of plasmatic cholesterol.

### Example 5

### Prebiotic activity of the extract

In order to verify the extract's prebiotic activity, a pilot fermentation with a 2 liter fermenter in a semi-continuous system under conditions of anaerobiosis was developed. The fermentation unit was equipped with control systems for the control of temperature (37 ± 0.1°C) and pH (6.1± 0.2) through the addition of NaOH 2M or H₂SO₄ 0.5M and stirring (100 rpm). The vessel and the medium were continuously insufflated with a mixture of anaerobic gas (80% N₂, 10% CO₂, 10% H₂) at 100 and 50 ml/min in order to maintain the state of anaerobiosis. The fermentation system was employed with the purpose of simulating the human intestine using the semi-continuous culture system inoculated with a pool of human feces. The principal groups of microorganisms in the human microbiota were monitored during fermentation processes for an evaluation of their effects. For semi-continuous fermentation, a complex culture medium similar to the one reported in the study by Zampa et al. (Zampa, A.; Silvi, S.; Fabiani, R.; Morozzi, G.; Orpianesi, C.; Cresci, A. Effects of different digestible carbohydrates on bile acid metabolism and SCFA production by human gut micro-flora grown in an in vitro semi-continuous culture, Anaerobe, 2004, 10, 19-26) enriched with fecal fluid that contained unknown components otherwise not present in the culture medium was used. Primary bile acids, colic and chenodeoxycholic acid (Sigma-Aldrich, Milano, Italy) were added to the ready-made culture medium in 0.6 g/l concentration.

Three semi-continuous fermentation processes were conducted: 1) with the addition of glucose to the medium as the sole source of carbohydrates (control group); 2) with the addition of inulin to the medium as the sole source of carbohydrates (positive control); 3) with the addition of lentil extract (*Extract A*) to the medium as the sole source of carbohydrates. Each fermentation process lasted 5 days and was repeated three times. The medium was Peptone Yeast Broth (PY) composed of: distilled water (1000 ml), Peptone 10g/I, Yeast extract 10g/I, with the addition of a saline solution (40 ml/l) and cysteine (0.5 g/l). The pH was brought to 7.2 with NaOH and then sterilized at 121 °C for 15 minutes (1 Bar). Following sterilization in the medium cooled at room temperature, glucose at 1% was added to the control group, inulin at 1% was added to the positive control group, and a quantity of lentil extract corresponding to a carbohydrate concentration of 1% was added to the fermentation test group. Furthermore, haemin (10 ml/l) and vitamin K (0.2 ml/l) were added to the fermentation media.

### Inoculation

The fermenter was inoculated with a pool of human feces obtained from volunteers in good health, without any gastrointestinal problems or having used antibiotics for at least one month prior to the sampling of their feces. Two grams of feces from each donor were weighed and placed in a sterile bag per stomacher and homogenized with the addition of a reducing saline solution (dilution 1:5). Following homogenization, the feces sample was divided into 9 bags of 5 ml capacity each, 1 per fermentation, and conserved at -80 °C.

### Fermentation process

Each fermentation process was inoculated with a pool of feces conserved at -80°C. The fermenter was filled with 1.2 I of culture medium that had been appropriately sterilized and inoculated the day before with 5 ml of inoculate formulated as described above. The fermentation process was run with the following parameters: DO₂ (Oxygen delivery), dissolved oxygen equal to 0%, pH at 6.6, temperature at 37°C, stirring at 100 rpm. The fermenter was left for 24h with the growth system set in "batch". Semi-continuous fermentation was started on the 2^{nd} day by inoculating with the use of a peristaltic pump, 6 feedings per day of 240 ml quantity each, and sampling was conducted by taking a corresponding 6 samples each day of the same volume, always prior to feeding. Every day, the first sampling was subjected to microbiological analysis. After 5 days, the fermentation process was terminated.

### Microbiological analysis conducted

Microbe counts were conducted by following the two-fold serial dilution, plate seeding method at elapsed time (0h), and after 24, 48, 72, and 96 hours from the start of the fermentation process, on selective culture media in conditions of aerobiosis and anaerobiosis at 37 °C for 24-72 h.

The following culture mediums were used for microbiological analysis: de Man, Rogosa, Sharpe agar (MRS) (Liofilchem^{®}, Roseto degli Abruzzi, Italy) for the *Lactobacillus* spp. genera count, Mannitol Salt agar (MSA) (Liofilchem^{®}) for the *Staphylococcus* spp. genera count, Columbia agar base + Hemin (1%) (bioMérieux, Marcy l'Etoile, France) for the total anaerobe count, MacConkey agar (Liofilchem^{®}) for the *Enterobacteriaceae* count, Beerens' agar medium (Silvi *et al.,* 1996; Silvi *et al.,* 2003) for the *Bifidobacteria* genera count, and Reinforced Clostridia Agar (OXOID) for the *Clostridium* spp. genera count.

### Results

The results regarding the counts of the principal groups of microorganisms of human intestinal microbiota in the three fermentation processes are provided in the table below (Table 2).

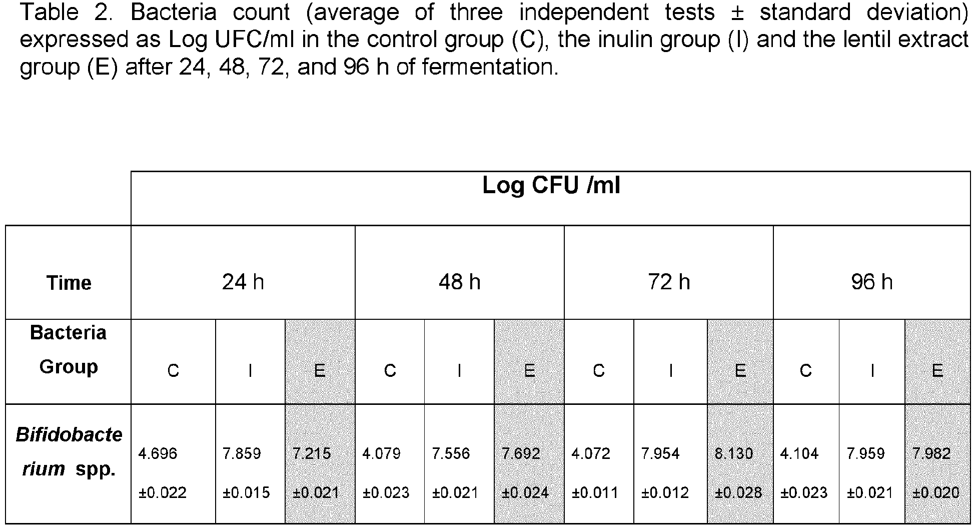

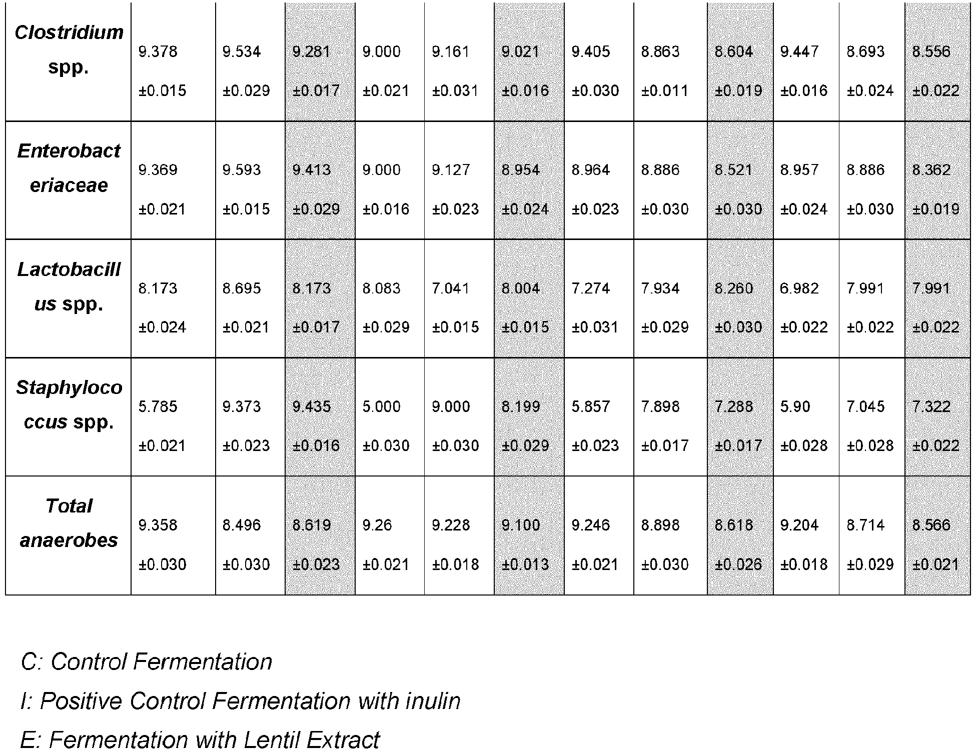

As a whole, the results demonstrate that inulin and lentil extract have the same effect on the main bacteria groups studied in the fermentation processes. *Enterobacteriaceae* behave in the same way in the presence of inulin as in the presence of lentil extract, and similar behavior was observed also in the control group. *Staphylococcus* increased in both the fermentation with lentil extract and with Inulin compared to the control group, whereas *Clostridium* bacteria decreased from 10⁹ to 10⁸ (UFC/ml) during fermentation in both the lentil extract and the inulin groups, even if to statistically negligible degree. Also worthy of note was the behavior of *Lactobacillus,* which tended to decrease in the control group but not during fermentation in either the inulin group or most importantly, in the lentil extract group, where the count remained high around 10⁸ (UFC/ml) after 96 h of fermentation. Another interesting result obtained regarded the total anaerobe count, which was significantly lower (Student t test, where p< 0.05) throughout the fermentation process with both inulin and lentil extract.

Lastly, as regards the *Bifidobacteria* group, statistical analysis applied to the results of the microbe count demonstrated a significantly higher increase in number during fermentation both in the lentil group and in the inulin group, confirming the lentil extract's capacity to exert *Bifidobacteria* generating action (see Figure 1 and Table 2).

More specifically, in regard to the count of bacterial groups considered beneficial, *Lactobacillus* showed stable performance in fermentation with inulin and lentil extract. The count observed for *Bifidobacteria* in the control group was low and stable at 10⁴ UFC/ml but increased in the inulin and lentil extract fermentation groups, the latter by up to 3 logarithms more than those of the control group, demonstrating in this way properties (including the stimulation of the growth of *Bifidobacteria*) that were previously recognized for inulin but undoubtedly newly demonstrated for the lentil extract.

Considering the results obtained and the inulin activity in *in vitro* fermentation, in the semi-continuous model, the effect of the lentil extract on the modulation of the principal groups of microorganisms of human intestinal microbiota is comparable to that of inulin, leading to the conclusion that the lentil extract analyzed possesses significant prebiotic and *Bifidobacteria* generating action.

Finally, the lentil extract possesses hypocholesterolemic action and prebiotic activity; in particular, the hypocholesterolemic effect shown in *in vivo* experiments and in the analysis of bile acids is powered by the prebiotic activity of extract that, acting as inulin, works not only as a prebiotic but contributes also to the cholesterol lowering effect. This synergic effect (hypocholesterolemic and prebiotic) results to be totally new for a lentil extract.

## Claims

1. A lentil (Lens culinaris L.) extract **characterized by** the presence of soyasaponin I in the range comprised between 50 and 300 mg kg-1 and of soyasaponin βg in the range comprised between 0.5 and 5 mg kg⁻¹ obtainable by a process **characterized by** the following steps:
a) adding a 70:30 mixture of water and ethanol to a ground lentil (Lens culinaris L.) sample, with the proviso that said mixture does not comprise ethylenediaminetetraacetic acid (EDTA);
b) stirring the obtained mixture;
c) filtering the obtained mixture;
d) concentrating the obtained mixture until complete evaporation of ethanol.

2. The extract according to claim 1 wherein said lentil (Lens culinaris L.) sample is of Colfiorito variety.

3. The extract according to claim 1 or 2 wherein step d) is carried out at a temperature of less than 30°C.

4. A lyophilized extract obtained by a process according to anyone of claims 1-3 comprising a further step of concentration and subsequent lyophilization of the obtained mixture.

5. The extract of anyone of claims 1-4 for use in the treatment of a disease selected from the group consisting of: cardiovascular diseases, such as myocardial infarction, type 2 diabetes, obesity and high blood pressure.

6. The extract of anyone of claims 1-4 for use for the maintenance of cholesterol level in a subject predisposed to develop hypercholesterolemia.

7. The extract of anyone of claims 1-4 for use as a prebiotic for the preservation and/or restoration of intestinal bacterial flora.

8. The use according to claim 7 wherein growth and/or activity of Bifidobacteria and Lactobacilli is promoted.

9. The extract of anyone of claims 1-4 for use in the prevention and/or in the treatment of gastrointestinal diseases and disorders wherein an alteration of the intestinal bacterial flora is present, wherein said disease or disorder is selected from irritable bowel syndrome, chronic inflammatory bowel diseases, diarrhea, dyspeptic forms, meteorism, diverticulosis, syndrome of bacterial overgrowth in the small intestine, alteration of the immune system and food allergies.

10. The extract of anyone of claims 1-4 for use in the treatment and/or prevention of intestinal dysbiosis.

11. The extract of anyone of claims 1-4 for use in the treatment of diseases and conditions wherein stimulation of the growth and/or activity of microbial species present in intestinal microbiota is beneficial for the health, for example diabetes, metabolic syndrome, obesity and allergy.

12. A nutraceutical, a functional food or a dietary supplement comprising the extract of anyone of claims 1-4, optionally in combination with dietary supplements, compounds or extracts with cholesterol lowering activity, compounds or extracts with prebiotic activity, such as inulin, fructo-oligosaccharides (FOS) and pectins, and/or compounds or extracts with probiotic activity.

13. The nutraceutical, functional food or dietary supplement according to claim 12 wherein said compound or extract with cholesterol lowering activity is selected from the group consisting of: phytostanols, phytosterols, fermented red rice, berberine and silymarin.

14. A pharmaceutical composition comprising the extract of anyone of claims 1-4.

## Patentansprüche

1. Linsen-(Lens culinaris L.-)Extrakt, **gekennzeichnet durch** das Vorhandensein von Sojasaponin I im Bereich zwischen 50 und 300 mg·kg⁻¹ und Sojasaponin βg im Bereich zwischen 0,5 und 5 mg·kg⁻¹, der durch ein Verfahren erhältlich ist, das durch die folgenden Schritte gekennzeichnet ist:
a) Hinzugeben eines 70:30-Gemischs aus Wasser und Ethanol zu einer gemahlenen Linsen-(Lens culinaris L.-)Probe, mit der Maßgabe, dass diese Mischung keine Ethylendiamintetraessigsäure (EDTA) enthält;
b) Rühren des erhaltenen Gemischs;
c) Filtern des erhaltenen Gemischs;
d) Konzentrieren des erhaltenen Gemischs bis zur vollständigen Verdampfung des Ethanols.

2. Extrakt nach Anspruch 1, wobei die Linsen-(Lens culinaris L.-)Probe von der Sorte Colfiorito ist.

3. Extrakt nach Anspruch 1 oder 2, wobei der Schritt d) bei einer Temperatur von weniger als 30 °C durchgeführt wird.

4. Lyophilisierter Extrakt, der durch ein Verfahren nach einem der Ansprüche 1 bis 3 erhalten wird, das einen weiteren Schritt des Konzentrierens und einer anschließenden Lyophilisierung des erhaltenen Gemischs umfasst.

5. Extrakt nach einem der Ansprüche 1 - 4 zur Verwendung bei der Behandlung einer Erkrankung, ausgewählt aus der Gruppe, bestehend aus: Herz-Kreislauf-Erkrankungen, wie z. B. einem Myokardinfarkt, Typ-2-Diabetes, Fettleibigkeit und Bluthochdruck.

6. Extrakt nach einem der Ansprüche 1 - 4 zur Verwendung für die Aufrechterhaltung eines Cholesterinspiegels bei einer Person, die zur Entwicklung einer Hypercholesterinämie prädisponiert ist.

7. Extrakt nach einem der Ansprüche 1 - 4 zur Verwendung als Präbiotikum für die Erhaltung und/oder Wiederherstellung der bakteriellen Darmflora.

8. Verwendung nach Anspruch 7, wobei das Wachstum und/oder die Aktivität von Bifidobakterien und Laktobazillen gefördert wird.

9. Extrakt nach einem der Ansprüche 1 - 4 zur Verwendung bei der Vorbeugung und/oder Behandlung von Magen-Darm-Erkrankungen und -Störungen, bei denen eine Veränderung der bakteriellen Darmflora vorliegt, wobei die Erkrankung oder Störung aus Reizdarmsyndrom, chronisch-entzündlichen Darmerkrankungen, Durchfall, dyspeptischen Formen, Meteorismus, Divertikulose, Syndrom der bakteriellen Überwucherung im Dünndarm, Veränderung des Immunsystems und Nahrungsmittelallergien ausgewählt ist.

10. Extrakt nach einem der Ansprüche 1 - 4 zur Verwendung bei der Behandlung und/oder Vorbeugung einer Darmdysbiose.

11. Extrakt nach einem der Ansprüche 1 - 4 zur Verwendung bei der Behandlung von Erkrankungen und Beschwerden, bei denen eine Stimulation des Wachstums und/oder der Aktivität von in der Darmflora vorhandenen mikrobiellen Spezies, wie z. B. Diabetes, einem metabolischen Syndrom, Fettleibigkeit und Allergien, für die Gesundheit vorteilhaft ist.

12. Nutrazeutikum, funktionelles Lebensmittel oder Nahrungsergänzungsmittel, umfassend den Extrakt nach einem der Ansprüche 1 - 4, gegebenenfalls in Kombination mit Nahrungsergänzungsmitteln, Verbindungen oder Extrakten mit cholesterinsenkender Wirkung, Verbindungen oder Extrakten mit präbiotischer Wirkung, wie z. B. Inulin, Fructo-Oligosaccharide (FOS) und Pektine, und/oder Verbindungen oder Extrakten mit probiotischer Wirkung.

13. Nutrazeutikum, funktionelles Lebensmittel oder Nahrungsergänzungsmittel nach Anspruch 12, wobei die Verbindung oder der Extrakt mit cholesterinsenkender Wirkung ausgewählt ist aus der Gruppe, bestehend aus: Phytostanolen, Phytosterolen, fermentiertem roten Reis, Berberin und Silymarin.

14. Pharmazeutische Zusammensetzung, die den Extrakt nach einem der Ansprüche 1 - 4 umfasst.

## Revendications

1. Extrait de lentille (Lens culinaris L.) **caractérisé par** la présence de saponine de soja I dans la gamme comprise entre 50 et 300 mg kg-1 et de saponine de soja βg dans la gamme comprise entre 0,5 et 5 mg kg-1 pouvant être obtenu par un procédé **caractérisé par** les étapes suivantes:
a) addition d'un mélange 70:30 d'eau et d'éthanol à un échantillon de lentille (Lens culinaris L.) broyé, à condition que ledit mélange ne comprenne pas d'acide éthylènediaminetétraacétique (EDTA);
b) agitation du mélange obtenu;
c) filtration du mélange obtenu;
d) concentration du mélange obtenu jusqu'à évaporation complète de l'éthanol.

2. Extrait selon la revendication 1 dans lequel ledit échantillon de lentille (Lens culinaris L.) est de variété Colfiorito.

3. Extrait selon la revendication 1 ou 2, dans lequel l'étape d) est réalisée à une température inférieure à 30°C.

4. Extrait lyophilisé obtenu par un procédé selon l'une quelconque des revendications 1 à 3 comprenant une étape supplémentaire de concentration et de lyophilisation ultérieure du mélange obtenu.

5. Extrait selon l'une quelconque des revendications 1 à 4 destiné à être utilisé dans le traitement d'une maladie choisie dans le groupe consistant en: les maladies cardiovasculaires, telles que l'infarctus du myocarde, le diabète de type 2, l'obésité et l'hypertension artérielle.

6. Extrait selon l'une quelconque des revendications 1 à 4 destiné à être utilisé pour le maintien du taux de cholestérol chez un sujet prédisposé à développer une hypercholestérolémie.

7. Extrait selon l'une quelconque des revendications 1 à 4 destiné à être utilisé comme prébiotique pour la préservation et/ou la restauration de la flore bactérienne intestinale.

8. Utilisation selon la revendication 7, dans laquelle la croissance et/ou l'activité des bifidobactéries et des lactobacilles est favorisée.

9. Extrait selon l'une quelconque des revendications 1 à 4 destiné à être utilisé dans la prévention et/ou le traitement de maladies et affections gastro-intestinales dans lesquelles une altération de la flore bactérienne intestinale est présente, dans lequel ladite maladie ou affection est choisie parmi le syndrome du côlon irritable, les maladies inflammatoires chroniques de l'intestin, la diarrhée, les formes dyspeptiques, le météorisme, la diverticulose, le syndrome de prolifération bactérienne dans l'intestin grêle, l'altération du système immunitaire et les allergies alimentaires.

10. Extrait selon l'une quelconque des revendications 1 à 4 destiné à être utilisé dans le traitement et/ou la prévention de la dysbiose intestinale.

11. Extrait selon l'une quelconque des revendications 1 à 4 destiné à être utilisé dans le traitement de maladies et d'affections dans lesquelles une stimulation de la croissance et/ou de l'activité d'espèces microbiennes présentes dans le microbiote intestinal est bénéfique pour la santé, par exemple le diabète, le syndrome métabolique, l'obésité et l'allergie.

12. Nutraceutique, aliment fonctionnel ou complément alimentaire comprenant l'extrait selon l'une quelconque des revendications 1 à 4, éventuellement en combinaison avec des compléments alimentaires, des composés ou des extraits à activité hypocholestérolémiante, des composés ou des extraits à activité prébiotique, tels que l'inuline, les fructooligosaccharides (FOS) et les pectines, et/ou des composés ou extraits à activité probiotique.

13. Nutraceutique, aliment fonctionnel ou complément alimentaire selon la revendication 12, dans lequel ledit composé ou extrait ayant une activité hypocholestérolémiante est choisi dans le groupe consistant en: les phytostanols, les phytostérols, le riz rouge fermenté, la berbérine et la silymarine.

14. Composition pharmaceutique comprenant l'extrait selon l'une quelconque des revendications 1 à 4.
